# EUROPEAN PATENT APPLICATION

(11) **EP 1 980 853 A1**
(43) Date of publication of application: **15.10.2008**
(21) Application number: 07007429.9
(22) Date of filing: 11.04.2007
(51) Int. Cl.: G01N 33/53, G01N 33/543, G01N 33/58

(54) **Method of preventing precipitation of a reactive substance-bound microparticle, and reagent containing the micro particle**

(71) Applicant: Alfresa Pharma Corporation, Osaka-shi, Osaka 540-8575 (JP)
(72) Inventor: Tanaka, Mutsumi, Suita-shi, Osaka 565-0817 (JP)
(74) Representative: Arth, Hans-Lothar

(57) **Abstract**

According to the present invention, precipitation of a reactive substance-bound microparticle can be prevented in a dispersion liquid, to make a concentration of the reactive substance-bound microparticle uniform in the dispersion liquid. The present invention provides a method of preventing precipitation of a reactive substance-bound microparticle. The method includes coexisting at least one selected from the group consisting of polyanion or its salt, dextran, cyclodextrin, polyethylene glycol, and glycerol, with the microparticle in a dispersion liquid.

## Description

### BACKGROUND OF THE INVENTION

### 1. Field of the Invention

The present invention relates to a method of preventing precipitation of a microparticle to which a reactive substance, such as an antibody or an antigen, is bound (hereinafter, referred to as a reactive substance-bound microparticle), and an immunoassay reagent which contains a reactive substance-bound microparticle. In particular, the present invention relates to a method of preventing precipitation of a reactive substance-bound microparticle, and an immunoassay reagent which contains a reactive substance-bound microparticle, for immunoassay that uses an antigen-antibody reaction in the field of clinical analysis.

### 2. Description of the Related Art

In recent years, in various analyses such as clinical analysis, a method of measuring substances in a biological sample by immune response, that is an immunoassay, has become widely used for automization and reduction in time of the measurement. Examples of immunoassay include RIA, EIA, immunonephelometry, latex agglutination, colloidal gold agglutination, and immunochromatography. Of these, an assay using a microparticle to which a reactive substance is bound (reactive substance-bound microparticle), such as latex agglutination or colloidal gold agglutination, does not require separation of the reaction mixture or washing, and thus is particularly suitable for automization and reduction in time of the measurement.

However, there are problems in the latex agglutination and colloidal gold agglutination. Since the reactive substance-bound microparticle is apt to precipitate, it is necessary to agitate to make the concentration of the microparticle uniform, for example, during use or measurement. Moreover, measurement error is prone to occur.

For example, two kinds of reagents are usually used in these methods. A first reagent mainly contains components that facilitate the dilution of the sample, the pre-treatment, or the main reaction of the sample. A second reagent contains a reactive substance-bound microparticle, which is the primary component mainly for the reaction. When an automated analyzer is used, first a sample and a first reagent are added into a reaction cell, and then, once a certain time has elapsed, a second reagent is added into the reaction cell, and the sample, the first reagent, and the second reagent are mixed to obtain a final reaction mixture, and then measurement is performed. In the case, once a reagent that contains the reactive substance-bound microparticle (a second reagent) has been applied to the analyzer, it was necessary to agitate the second reagent at interval so as to make the concentration uniform.

To date, there have been no attempt to prevent precipitation of the reactive substance-bound microparticle. A first reagent, a second reagent, or a final reaction mixture has been only improved with the aim of increasing the stability of the reactivity of the microparticle or enhancing the reaction (for example, see Japanese Laid-Open Patent Publication No. 11-101799 and Japanese Laid-Open Patent Publication No. 6-213891).

Japanese Laid-Open Patent Publication No. 11-101799 discloses that a method for stabilizing the reactivity of sensitized colloidal metal. The method includes adding one or two or more substances selected from metal ions selected from calcium ion, magnesium ion, and barium ion, and dextran sulfate, cholic acid, deoxycholic acid, xanthurenic acid, and salts thereof to a solution that contains a sensitized colloidal metal.

Japanese Laid-Open Patent Publication No. 6-213891 discloses that a polyanion or its salt having an average molecular weight of approximately 20,000 or more can be included as a reaction enhancer at 0.4 to 2.5 (W/W)% in a liquid (a final reaction mixture) including a substance to be analyzed and a colloidal gold particle to which an antibody (or antigen) for the substance is bound.

### SUMMARY OF THE INVENTION

In view of above, in order to achieve a reduction in measured value error, there has been a desire for a technology capable of preventing precipitation of the reactive substance-bound microparticle in order to make a concentration of the microparticle in a reagent or reaction mixture uniform over an extended period of time. In particular, when failing to perform the agitation operation after the reagent has been applied to automated analyzer, measurement error may occur, thereby leading to a mistake of clinical decision. It is likely important to develop a method of preventing precipitation of a reactive substance-bound microparticle in the dispersion and a reagent that utilizes the same.

Thus, it is an object of the present invention to provide a method of preventing precipitation of a reactive substance-bound microparticle in a dispersion of the microparticle. Another object of the present invention is to provide a reagent capable of reducing measurement error, which does not require any additional agitation for the sake of dispersion of the microparticle after the reagent has been allowed to stand or applied to an automated immuno-analyzer.

As a result of in-depth research to solve the above-described problem, the inventors found that at least one compound selected from the group consisting of polyanion or its salt, dextran, cyclodextrin, polyethylene glycol, and glycerol can be included in a dispersion of the reactive substance-bound microparticle to prevent precipitation of the reactive substance-bound microparticle and to make a concentration of the reactive substance-bound microparticle in the dispersion uniform over an extended period of time, and thus arrived at the invention.

The present invention provides a method of preventing precipitation of a reactive substance-bound microparticle, and the method includes coexisting at least one selected from the group consisting of polyanion or its salt, dextran, cyclodextrin, polyethylene glycol, and glycerol, with the microparticle in a dispersion liquid.

In one embodiment, the dispersion liquid contains polyanion or its salt at 0.3 to 5 percent by weight, dextran at 0.1 to 5 percent by weight, cyclodextrin at 0.1 to 5 percent by weight, polyethylene glycol at 0.3 to 5 percent by weight, or glycerol at 5 to 40 percent by weight.

In another embodiment, the polyanion or its salt is a water-soluble, sulfate group-containing polymer compound.

In yet another embodiment, the polyanion is dextran sulfate or heparin.

In still another embodiment, the reactive substance-bound microparticle is a colloidal gold particle to which an antibody or antigen is bound.

A reagent of the present invention includes a reactive substance-bound microparticle and at least one compound selected from the group consisting of polyanion or its salt, dextran, cyclodextrin, polyethylene glycol, and glycerol, wherein precipitation of the microparticle is prevented.

In one embodiment, the reagent contains polyanion or its salt at 0.3 to 5 percent by weight, dextran at 0.3 to 5 percent by weight, cyclodextrin at 0.1 to 5 percent by weight, polyethylene glycol at 0.5 to 5 percent by weight, or glycerol at 5 to 40 percent by weight.

In another embodiment, the polyanion or its salt is a water-soluble, sulfate group-containing polymer compound.

In yet another embodiment, the polyanion is dextran sulfate or heparin.

In still another embodiment, the reactive substance-bound microparticle is a colloidal gold particle to which an antibody or antigen is bound.

A reagent kit of the invention includes the reagent of the invention.

An automated immunoassay method of the present invention includes combining the reagent of the invention with a sample.

According to the present invention, precipitation of a reactive substance-bound microparticle can be prevented. Consequently, it becomes possible to provide a reagent in which precipitation of the microparticle prevented from occuring. The reagent of the present invention allows the concentration of the microparticle uniform over an extended period of time. For example, if the reagent is used for an immunoassay, then reactive substance-bound microparticles therein can be added uniformly to final reaction mixture without agitating the reagent on measuring. Therefore, stably measured values with little error can be provided.

Specifically, if the reagent containing the reactive substance-bound microparticle of the present invention is applied to an automated analyzer to measure continuously for an extended period of time or at interval, accurately measured values with little error can be provided over an extended period of time. The present invention eliminates the task of agitating the reagent containing the microparticles at a batch of measurement or at interval in order to make the concentration of the microparticle uniform in the dispersion liquid, which previously required.

Thus, it is possible to not only eliminate necessity for any complicated agitation operations, which imposed a large burden on user, but also prevent measurement error and mistake of clinical decisions due to failing to perform the agitation operation.

### BRIEF DESCRIPTION OF THE DRAWINGS

Fig. 1 is a graph showing the variation in the measured values from the measurement over time using a predetermined concentration of cystatin C.
Fig. 2 is a graph showing the variation in the measured values from the measurement over time using a predetermined concentration of ferritin.

### DESCRIPTION OF THE PREFERRED EMBODIMENTS

### 1. Method of preventing precipitation of a reactive substance-bound microparticle

The method for preventing precipitation of a reactive substance-bound microparticle of the present invention includes coexisting at least one selected from the group consisting of polyanion or its salt, dextran, cyclodextrin, polyethylene glycol, and glycerol, with the microparticle in a dispersion liquid.

The reactive substance-bound microparticle used in the method of the present invention includes any microparticles that can be used for an immunoassay reagent, preferably latex particles or colloidal metal particles. As the colloidal metal particles, in general, colloidal gold particles can be conveniently used and colloidal gold particles are preferably used in the present invention. Colloidal gold particles are commercially available, or colloidal gold particles that can be prepared by any methods usually carried out by those skilled in the art, such as reducing gold chloride with sodium citrate. The particle diameter of the colloidal gold particles is in the range of usually 5 nm to 100 nm, and preferably 30 nm to 60 nm.

As a substance that is bound to the microparticle, any substances capable of binding specifically to a substance to be measured can be used. Examples of the substance bound to the microparticle include any substances having binding affinity, such as an antibody, antigen, receptor, lecithin, deoxyribonucleic acid (DNA), or ribonucleic acid (RNA).

In the method of the present invention, polyanion or its salt, dextran, cyclodextrin, polyethylene glycol, or glycerol can be used. Hereinafter, the above-mentioned compounds are also collectively referred to as a precipitation preventing substance. As the precipitation preventing substance, a single compound may be used, or two or more different compounds may be used in combination.

Examples of polyanion, one of the precipitation preventing substance, include dextran sulfate, heparin, polystyrene sulfonate, hyaluronic acid, and chondroitin sulfate. Examples of salt of polyanion include dextran sulfate sodium and heparin sodium. Among others, a water-soluble, sulfate group-containing polymer compound is preferably used in view of a high precipitation preventing effect. Examples of such a compound include dextran sulfate, heparin, dextran sulfate sodium, and heparin sodium.

The method of the present invention includes coexisting the precipitation preventing substance with the reactive substance-bound microparticle in a dispersion liquid. There are no particular limitations to the means by which coexistence is achieved. For example, the precipitation preventing substance can be added to a dispersion of the reactive substance-bound microparticle, the reactive substance-bound microparticle can be added to the precipitation preventing substance or to a dilution of the precipitation preventing substance in a dispersion solvent, or the reactive substance-bound microparticle can be mixed with the precipitation preventing substance and then the mixture added to a dispersion solvent. There are no particular limitations regarding the dispersion solvent. As the dispersion solvent, water or a buffer solution as discussed later can be used.

In the method of the invention, the concentration of the reactive substance-bound microparticle in the dispersion liquid can be a concentration usually used in the art.

In the method of the invention, the concentration of the precipitation preventing substance in the dispersion liquid can be suitably determined depending on the type of the precipitation preventing substance. For example, when polyanion or its salt can be used, preferably the concentration in the dispersion liquid is 0.3 to 5 percent by weight (wt%), more preferably 0.5 to 2 wt%, and even more preferably 0.7 to 1.1 wt%. When dextran or cyclodextrin can be used, preferably the concentration in the dispersion liquid is 0.1 to 5 wt%, more preferably 0.2 to 1.8 wt%, and even more preferably 0.5 to 1.5 wt%. When polyethylene glycol can be used, preferably the concentration in the dispersion liquid is 0.3 to 5 wt%, and more preferably 0.5 to 3 wt%. When glycerol can be used, preferably the concentration in the dispersion liquid is 5 to 40 wt%, and more preferably 10 to 35 wt%.

According to the method of the invention, precipitation of the reactive substance-bound microparticle can be prevented by the coexistence of a precipitation preventing substance with a reactive substance-bound microparticle in a dispersion liquid. Consequently, according to the method, the microparticle can be uniform present in the dispersion liquid over an extended period of time.

### 2. Reagent containing a reactive substance-bound microparticle and a precipitation preventing substance

The reagent of the invention contains a reactive substance-bound microparticle and a precipitation preventing substance, and optionally buffer, succharide, sugar alcohol, albumin, sodium chloride, antiseptic, or other additives. The above reagent allows precipitation of the reactive substance-bound microparticle to be prevented, so that the concentration of the microparticle can be uniform over an extended period of time. The reagent of the invention is usually a liquid form, and can be used as a reagent that contains a reactive substance-bound microparticle, and in particular as a second reagent for immunoassay usually used in the art.

There are no particular limitations regarding the concentrations of the reactive substance-bound microparticle, and the precipitation preventing substance in the reagent of the invention. In view of increasing an effect of preventing precipitation of the reactive substance-bound microparticle, these are preferably within the range of concentrations as adopted in the method explained above.

Examples of buffer that can be contained in the reagent of the invention include phosphate buffer solution; tris-hydrochloride buffer solution; succinate buffer solution; and Good's buffer such as glycylglycine, MES (2-(N-Morpholino)ethanesulfonic acid), HEPES (N-2-hydroxyethyl-piperazine-N'-ethanesulfonic acid), TES (N-tris(hydroxymethyl)methyl-2-aminoethanesulfonic acid), MOPS (3-(N-Morpholino)propanesulfonic acid), PIPES (piperazine-1,4-bis(2-ethanesulfonic acid)), and Bis-Tris (bis(2-hydroxyethyl)iminotris(hydroxymethyl)methane). The buffer is preferably contained to make a pH of 5 to 9 or at 1 to 100 mM in the reagent.

Examples of saccharide or sugar alcohol that can be contained in the reagent of the invention include glucose, mannose, saccharose, lactose, maltose, mannitol, and sorbitol. The saccharide or sugar alcohol is preferably at 0.01 to 10 wt% in the reagent.

Examples of albumin that can be contained in the reagent of the invention include bovine serum albumin (BSA). The albumin is preferably at 0.001 to 1 wt% in the reagent.

Examples of antiseptic that can be contained in the reagent of the invention include sodium azide. The antiseptic is preferably at 0.01 to 0.5 wt% in the reagent.

Examples of other additives that can be included in the reagent of the invention include Tween 20, polyethylene glycol lauryl ether, 5-bromosalicylic acid, sodium salicylate, sodium benzoate, sodium benzenesulfonate, phenol, and thymol.

As described above, the reagent of the invention allows the precipitation of the reactive substance-bound microparticle to be prevented and the concentration of the microparticle can be uniform in the reagent over an extended period of time. Consequently, for example, using the reagent for an immunoassay, reactive substance-bound microparticles in the reagent can be uniformly added to final reaction mixture without agitating the reagent on measuring. Therefore, stably measured values with little error can be provided.

### 3. Reagent Kit

The reagent kit of the invention includes a reagent (the reagent of the invention), that contains a reactive substance-bound microparticle and a precipitation preventing substance, as mentioned above. The reagent kit can be used for immunoassay, for example, using latex agglutination or colloidal gold agglutination, in particular, for automated immunoassay.

When the reagent kit of the invention is used as a reagent kit for immunoassay, the kit usually includes a first reagent, the reagent of the present invention (a second reagent), and, as necessary, a reference standard for creating the calibration curve. The first reagent can mainly contain components that facilitate the dilution of the sample, the pre-treatment, or the main reaction of the sample.

The reagent kit of the invention can be used to measure any substances in a sample. A reactive substance bound to the microparticle depends on the substance to be measured. Examples of substances capable of be measured (substances to be measured) include a protein, such as albumin, hemoglobin, hemoglobin A1c, myoglobin, transferrin, lactoferrin, cystatin C, ferritin, α-fetoprotein, carcinoembryonic antigen, CA19-9, prostate specific antigen, C-reactive protein (CRP), fibrin degradation product (FDP), pepsinogens I and II, and collagen; a lipoprotein, such as high density lipoprotein, low density lipoprotein, and very low density lipoprotein; a nucleic acid such as deoxyribonucleic acid and ribonucleic acid; an enzyme, such as alkaline phosphatase, lactate dehydrogenase, lipase, and amylase; immunoglobulins such as IgG, IgM, IgA, IgD, and IgE; an antigen or antibody for an infectious disease, such as hepatitis virus B, hepatitis virus C, human immunodeficiency virus, Helicobacter pylori, and antibodies therefor; drugs such a haloperidol and bromperidol; and a hormone, such as sex hormone.

Examples of sample that contains a substance to be measured include a biological sample, such as blood, plasma, serum, urine, feces (in suspension), cerebrospinal fluid, and peritoneal fluid, and a sample collected from the environment or extract therefrom.

### 4. Automated immunoassay method

The automated immunoassay method of the invention can be carried out using the reagent of the invention. Therefore, using the automated immunoassay method, reactive substance-bound microparticles in the reagent can be uniformly added to final reaction mixture without agitating the reagent on measuring, and thus stably measured values with little error can be provided. This is because that precipitation of the reactive substance-bound microparticle is prevented in the reagent, and the concentration of the microparticle can be uniformly present over an extended period.

Specifically, the automated immunoassay method of the invention includes applying a sample, a first reagent, and the reagent of the invention (a second reagent) to an automated analyzer, and automatically combining the first reagent and subsequently the second reagent with the sample. A ratio between the first reagent and the second reagent can be appropriately determined. Preferably, the reagent of the invention (a second reagent) is diluted by a dilution factor of 2 to 9 times, more preferably 3 to 5 times.

Using the automated immunoassay method of the invention, even when measuring continuously over an extended time or at interval, accurately measured values with little error can be provided over an extended period of time. The automated immunoassay method of the invention eliminates the task of agitating the reagent containing the microparticles at a batch of measurement or at interval in order to make the concentration of the microparticle uniform in the dispersion liquid, which previously required.

### Examples

Hereinafter, the invention is described in specific detail through Examples, but the invention is not limited to these Examples. In the Examples below, unless specifically noted otherwise, % refers to the weight/volume % (W/V%).

### Example 1: Preparation of a colloidal gold solution

Two milliliter of a 10% gold chloride solution was added to 1 L of 95°C distilled water while agitating, after one minute, 10 mL of a 2% sodium citrate solution was added, and after agitating for 20 minutes, cooled to 30°C. After cooling, the pH was adjusted to 7.1 with 0.1% potassium carbonate solution.

### Example 2: Preparation of first reagent for measuring cystatin C

A first reagent for measuring cystatin C was prepared by adding about 1.0 to 2.5% polyethylene glycol as a reaction enhancer to a solution containing 5% sodium chloride, 0.2% EDTA, 0.2% sodium alkylphenyldisulfonate salt, and 0.35% polyoxyethylene lauryl ether in 0.5 M Bis-Tris (pH 6.7).

### Example 3: Preparation of anti-cystatin C antibody bound colloidal gold reagent (second reagent)

Anti-cystatin C antibody (Dako Japan) was diluted with 10 mM HEPES (pH 7.1) containing 0.05% sodium azide to obtain a solution that contains 50 µg/mL of anti-cystatin C antibody, and hereinafter, referred to as an anti-cystatin C antibody solution. Then, 100 mL of the anti-cystatin C antibody solution was added to approximately 1 L of the colloidal gold solution prepared in Example 1, and then incubated for two hours under refrigeration with agitation. And then, 110 mL of a solution containing 5.46% mannitol, 0.5% BSA, and 0.05% sodium azide in 10 mM HEPES (pH 7.1) was added, and then incubated for 90 minutes at 37°C with agitation. The resulatant was centrifuged at 8000 rpm for 40 minutes to remove a supernatant, and then approximately 1 L of a solution containing 3% mannitol, 0.1% BSA, and 0.05% sodium azide in 5 mM HEPES (pH 7.5) (solution A) was added, to disperse the antibody-bound colloidal gold particles. Furthermore, the dispersion was centrifuged at 8000 rpm for 40 minutes to remove a supernatant, and the antibody-bound colloidal gold particles were dispersed with the solution A for a total volume of 210 mL, to obtain an anti-cystatin C antibody-bound colloidal gold reagent (second reagent).

### Example 4: Examination of the effect for adding glycerol or polyethylene glycol

### (1) Measurement of a sample (sample 1) with cystatin C at approximately 8 mg/L

To the anti-cystatin C antibody-bound colloidal gold reagent (second reagent), glycerol was added at a concentration of 33% to prepare a glycerol-added second reagent.

To a Hitachi 7070 automated analyzer, a sample (sample 1) with cystatin C at approximately 8 mg/L, a first reagent prepared in Example 2, and a glycerol-added second reagent were applied, and immediately after the application, absorbance change was determined under the following measurement condition.

### Measurement condition:

First, 240 µL of the first reagent were added to 3 µL of sample 1 and incubated at 37°C for approximately 5 minutes. Then 60 µL of a glycerol-added second reagent was added and reacted at 37°C. Subsequently, two-point measurement was performed at a dominant wavelength of 546 nm and a secondary wavelength of 660 nm from photometry points 18 to 31, and absorbance change between two points was determined. The absorbance change was determined for each of the case in which the second reagent was added without agitation (absorbance change without agitation) and the case in which the second reagent was added with agitation (absorbance change with agitation).

One day after the application, the absorbance change (for each without agitation and with agitation) was also determined in the same manner as above.

The difference was calculated from the absorbance change without agitation and the absorbance change with agitation for each of "immediately after application" and "after one day", and such a difference is, hereinafter, referred to as "the difference in the measured values". The results are shown in Table 1.

Further, when a second reagent that contains polyethylene glycol (PEG) at 2% (a PEG-added second reagent), a second reagent that contains glycerol at 33% and PEG at 2% (a mixture-added second reagent), or a second reagent without additional was used in place of the glycerol-added second reagent, the absorbance change was determined and the difference in the measured values was calculated in the same manner as above. The results are shown together in Table 1.

**Table 1**

| Sample | Second reagent | | Absorbance change (Δ Absorbance) | |
|---|---|---|---|---|
| | Precipitation preventing substance | Condition | Immediately after application | After one day |
| Sample 1 (Cystatin C: 8mg/L) | Containing glycerol at 33% | With agitation before measurement^{*1} | 0.7870 | 0.8028 |
| | | Without agitation | 0.7870 | 0.7814 |
| | | Difference in the measured values (With agitation - without agitation) | 0.0000 | 0.0214 |
| | Containing PEG at 2.0% | With agitation before measurement^{*1} | 0.7704 | 0.7905 |
| | | Without agitation | 0.7704 | 0.7648 |
| | | Difference in the measured values (With agitation - without agitation) | 0.0000 | 0.0257 |
| | Containing glycerol at 33% and PEG at2.0% | With agitation before measurement^{*1} | 0.7991 | 0.8135 |
| | | Without agitation | 0.799 | 0.8061 |
| | | Difference in the measured values (With agitation - without agitation) | 0.0000 | 0.0074 |
| | No additional (Control) | With agitation before measurement^{*1} | 0.8384 | 0.8487 |
| | | Without agitation | 0.8384 | 0.7674 |
| | | Difference in the measured values (With agitation - without agitation) | 0.0000 | 0.0813 |

| | | | | |
|---|---|---|---|---|
| *1···A second reagent is agitated before measuring. | | | | |

### (2) Measurement of a sample (sample 2) with cystatin C at approximately 4 mg/L

Using a sample (sample 2) with cystatin C at approximately 4 mg/L instead of sample 1, the difference in the measured values was calculated in the same manner as above (1). The results are shown in Table 2.

**Table 2**

| Sample | Second reagent | | Absorbance change (Δ Absorbance) | |
|---|---|---|---|---|
| | Precipitation preventing substance | Condition | Immediately after application | After one day |
| Sample 2 (Cystatin C: 4mg/L) | Containing glycerol at 33% | With agitation before measurement^{*1} | 0.5359 | 0.5525 |
| | | Without agitation | 0.5359 | 0.5296 |
| | | Difference in the measured values (With agitation - without agitation) | 0.0000 | 0.0229 |
| | Containing PEG at 2.0% | With agitation before measurement^{*1} | 0.5493 | 0.5723 |
| | | Without agitation | 0.5493 | 0.5415 |
| | | Difference in the measured values (With agitation - without agitation) | | |
| | Containing glycerol at 33% and PEG at2.0% | With agitation before measurement^{*1} | 0.5577 | 0.5676 |
| | | Without agitation | 0.5577 | 0.5676 |
| | | Difference in the measured values (With agitation - without agitation) | 0.0000 | 0.0000 |
| | No additional (Control) | With agitation before measurement^{*1} | 0.5763 | 0.5934 |
| | | Without agitation | 0.5763 | 0.5278 |
| | | Difference in the measured values (With agitation - without agitation) | 0.0000 | 0.0656 |

| | | | | |
|---|---|---|---|---|
| *1···A second reagent is agitated before measuring. | | | | |

### (3) Measurement of a sample (sample 3) with cystatin C at approximately 1 mg/L

Using a sample (sample 3) with cystatin C at approximately 1 mg/L instead of sample 1, the difference in the measured values was calculated in the same manner as above (1). The results are shown in Table 3.

**Table 3**

| Sample | Second reagent | | Absorbance change (Δ Absorbance) | |
|---|---|---|---|---|
| | Precipitation preventing substance | Condition | Immediately after application | After one day |
| Sample 3 (Cystatin C: 1 mg/L) | Containing glycerol at 33% | With agitation before measurement^{*1} | 0.1185 | 0.1245 |
| | | Without agitation | 0.1185 | 0.1173 |
| | | Difference in the measured values (With agitation - without agitation) | 0.0000 | 0.0072 |
| | Containing PEG at 2.0% | With agitation before measurement^{*1} | 0.1247 | 0.1296 |
| | | Without agitation | 0.1247 | 0.1166 |
| | | Difference in the measured values (With agitation - without agitation) | 0.0000 | 0.0130 |
| | Containing glycerol at 33% and PEG at2.0% | With agitation before measurement^{*1} | 0.1227 | 0.1281 |
| | | Without agitation | 0.1227 | 0.1279 |
| | | Difference in the measured values (With agitation - without agitation) | 0.0000 | 0.0002 |
| | No additional (Control) | With agitation before measurement^{*1} | 0.1218 | 0.1268 |
| | | Without agitation | 0.1218 | 0.1088 |
| | | Difference in the measured values (With agitation - without agitation) | 0.0000 | 0.0180 |

| | | | | |
|---|---|---|---|---|
| *1···A second reagent is agitated before measuring. | | | | |

It is clear from the results in Tables 1 to 3 that a second reagent to which a precipitation preventing substance (glycerol, polyethylene glycol, or a mixture of glycerol and polyethylene glycol) was added had a significantly smaller difference in measured values after one day, compared to that to which no precipitation preventing substance was added. Using a second reagent to which no precipitation preventing substance was added, one day after, the antibody-bound colloidal gold particles would precipitate due to gravity, thus the concentration of the particle would become diluted at the upper layer portion of the reagent. Consequently, in the automated analyzer, the reagent is added into the reaction cell from the upper layer region, where the concentration of the particle has become diluted. As the result, unless the reagent is agitated immediately prior to measurement, the amount of the antibody-bound colloidal gold particles directly involved in the reaction would be reduced, significantly reducing the absorbance change. For this reason, after standing undisturbed for one day, a large difference was seen in the absorbance changes between when the antibody-bound colloidal gold reagent was agitated before measuring and when the antibody-bound colloidal gold reagent was not agitated before measuring, even though the same sample was used for measurement.

From this it was found that by adding glycerol and/or polyethylene glycol to the antibody-bound colloidal gold reagent, the precipitation of the antibody-bound colloidal gold particles when stored undisturbed could be prevented and the uniformity of the antibody-bound colloidal gold reagent could be maintained.

### Example 5: Examination of the effect for adding dextran or dextran sulfate sodium

### (1) Measurement of a sample (sample 1) with cystatin C at approximately 8 mg/L

To the anti-cystatin C antibody-bound colloidal gold reagent (second reagent), dextran was added at a concentration of 1% to prepare a dextran-added second reagent.

Using a dextran-added second reagent in place of the glycerol-added second reagent, the absorbance change was determined for "after three days" in place of "after one day" and the difference in the measured values was calculated in the same manner as in (1) of Example 4. The results are shown together in Table 4.

Further, using a second reagent that contains dextran sulfate sodium at 1% (dextran sulfate-added second reagent), or the second reagent without additional in place of the dextran-added second reagent, the absorbance change was determined and the difference in the measured values was calculated in the same manner as above. The results are shown together in Table 4.

**Table 4**

| Sample | Second reagent | | Absorbance change (Δ Absorbance) | |
|---|---|---|---|---|
| | Precipitation preventing substance | Condition | Immediately after application | After three days |
| Sample 1 (Cystatin C: 8mg/L) | Containing dextran at 1.0% | With agitation before measurement^{*1} | 0.9040 | 0.9112 |
| | | Without agitation | 0.9040 | 0.8856 |
| | | Difference in the measured values (With agitation - without agitation) | 0.0000 | 0.0256 |
| | Containing dextran sulfate at 1.0% | With agitation before measurement^{*1} | 0.9368 | 0.9433 |
| | | Without agitation | 0.9368 | 0.9445 |
| | | Difference in the measured values (With agitation - without agitation) | 0.0000 | -0.0012 |
| | No additional (Control) | With agitation before measurement^{*1} | 0.9374 | 0.9458 |
| | | Without agitation | 0.9374 | 0.9088 |
| | | Difference in the measured values (With agitation - without agitation) | 0.0000 | 0.0370 |

| | | | | |
|---|---|---|---|---|
| *1···A second reagent is agitated before measuring. | | | | |

### (2) Measurement of a sample (sample 2) with cystatin C at approximately 4 mg/L

Using a sample (sample 2) with cystatin C at approximately 4 mg/L instead of sample 1, the difference in the measured values was calculated in the same manner as in (1) of Example 5. The results are shown in Table 5.

**Table 5**

| Sample | Second reagent | | Absorbance change (Δ Absorbance) | |
|---|---|---|---|---|
| | Precipitation preventing substance | Condition | Immediately after application | After three days |
| Sample 2 (Cystatin C: 4mg/L) | Containing dextran at 1.0% | With agitation before measurement*¹ | 0.5247 | 0.5367 |
| | | Without agitation | 0.5247 | 0.5173 |
| | | Difference in the measured values (With agitation - without agitation) | 0.0000 | 0.0194 |
| | Containing dextran sulfate at 1.0% | With agitation before measurement*¹ | 0.5784 | 0.5864 |
| | | Without agitation | 0.5784 | 0.5821 |
| | | Difference in the measured values (With agitation - without agitation) | 0.0000 | 0.0043 |
| | No additional (Control) | With agitation before measurement*¹ | 0.5716 | 0.5837 |
| | | Without agitation | 0.5716 | 0.5535 |
| | | Difference in the measured values (With agitation - without agitation) | 0.0000 | 0.0302 |

| | | | | |
|---|---|---|---|---|
| *1···A second reagent is agitated before measuring. | | | | |

It is clear from the results in Tables 4 and 5 that a second reagent to which a precipitation preventing substance (dextran or dextran sulfate sodium) was added had a significantly smaller difference in measured values after three days, compared to that to which no precipitation preventing substance was added. From this it was found that by adding a precipitation preventing substance (dextran or dextran sulfate sodium) to the antibody-bound colloidal gold reagent, the precipitation of the antibody-bound colloidal gold particles when stored undisturbed could be prevented and the uniformity of the antibody-bound colloidal gold reagent could be maintained.

### Example 6: Examination of the effect for adding heparin sodium

### (1) Measurement of a sample (sample 1) with cystatin C at approximately 8 mg/L

To the anti-cystatin C antibody-bound colloidal gold reagent (second solution), heparin sodium was added at a concentration of 1% to prepare a heparin-added second reagent.

Using a heparin-added second reagent in place of the glycerol-added second reagent, and the absorbance change was determined for "after two days" in place of "after one day" and the difference in the measured values was calculated in the same manner as in (1) of Example 4. The results are shown in Table 6.

**Table 6**

| Sample | Second reagent | | Absorbance change (Δ Absorbance) | |
|---|---|---|---|---|
| | Precipitation preventing substance | Condition | Immediately after application | After two days |
| Sample 1 (Cystatin C: 8mg/L) | Containing heparin at 1.0% | With agitation before measurement^{*1} | 0.8382 | 0.8369 |
| | | Without agitation | 0.8382 | 0.8163 |
| | | Difference in the measured values (With agitation - without agitation) | 0.0000 | 0.0206 |
| | No additional (Control) | With agitation before measurement^{*1} | 0.8330 | 0.8607 |
| | | Without agitation | 0.8330 | 0.7642 |
| | | Difference in the measured values (With agitation - without agitation) | 0.0000 | 0.0965 |

| | | | | |
|---|---|---|---|---|
| *1···A second reagent is agitated before measuring. | | | | |

### (2) Measurement of a sample (sample 2) with cystatin C at approximately 4 mg/L

Using a sample (sample 2) with cystatin C at approximately 4 mg/L instead of sample 1, the difference in the measured values was calculated in the same manner as (1) of Example 6. The results are shown in Table 7.

**Table 7**

| Sample | Second reagent | | Absorbance change (Δ Absorbance) | |
|---|---|---|---|---|
| | Precipitation preventing substance | Condition | Immediately after application | After two days |
| Sample 2 (Cystatin C: 4mg/L) | Containing heparin at 1.0% | With agitation before measurement^{*1} | 0.5156 | 0.5078 |
| | | Without agitation | 0.5156 | 0.5083 |
| | | Difference in the measured values (With agitation - without agitation) | 0.0000 | -0.0005 |
| | No additional (Control) | With agitation before measurement^{*1} | 0.5109 | 0.5156 |
| | | Without agitation | 0.5109 | 0.4607 |
| | | Difference in the measured values (With agitation - without agitation) | 0.0000 | 0.0549 |

| | | | | |
|---|---|---|---|---|
| *1···A second reagent is agitated before measuring. | | | | |

It is clear from the results in Tables 6 and 7 that a second reagent to which a precipitation preventing substance (heparin sodium) was added had a significantly smaller difference in measured values after two days, compared to that to which no precipitation preventing substance is added. From this it was found that by adding a precipitation preventing substance (heparin sodium) to the antibody-bound colloidal gold reagent, the precipitation of the antibody-bound colloidal gold particles when stored undisturbed could be prevented and the uniformity of the antibody-bound colloidal gold reagent could be maintained.

### Example 7: Effect for adding dextran sulfate sodium (Variations over time)

### (1) Measurement of a sample (sample 1) with cystatin C at approximately 8 mg/L

To the anti-cystatin C antibody-bound colloidal gold reagent (second reagent), dextran sulfate sodium was added at a concentration of 1% to prepare a dextran sulfate-added second reagent.

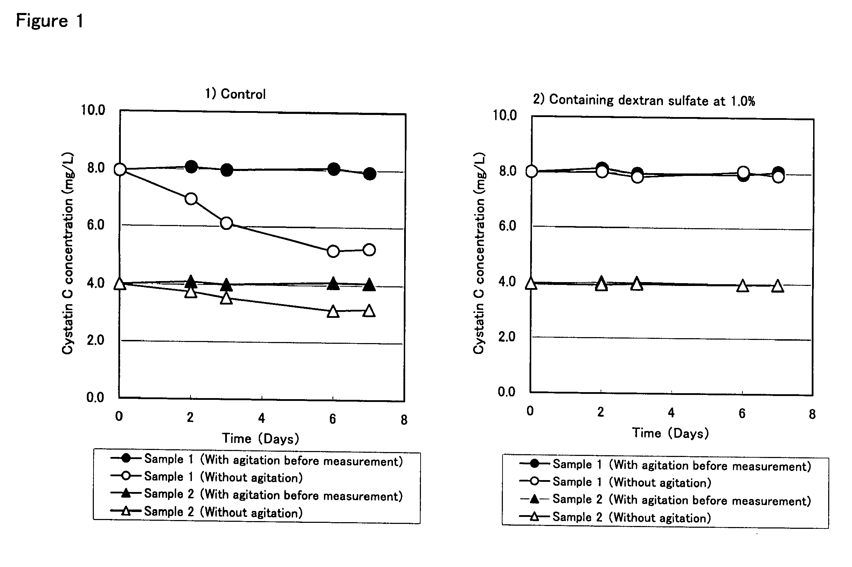

Using a dextran sulfate-added second reagent in place of the glycerol-added second reagent, the absorbance change was determined for each of "after two days", "after three days", "after six days", and "after seven days" in place of "after one day" in the same manner as in (1) of Example 4. A measured value of the cystatin C concentration was calculated from the absorbance change determined above using a calibration curve created with a standard solution with a defined concentration. The results are shown in Table 8 and Fig. 1.

**Table 8**

| Sample | Second reagent | | Measured value of cystatin C concentration (mg/L) | | | | |
|---|---|---|---|---|---|---|---|
| | Precipitation preventing substance | Condition | Immediately after application | After two days | After three days | After six days | After seven days |
| Sample 1 (Cystatin C: 8mg/L) | Containing dextran sulfate at 1.0% | With agitation before measurement^{*1} | 7.99 | 8.14 | 7.96 | 7.94 | 8.05 |
| | | Without agitation | 7.99 | 8.02 | 7.84 | 8.05 | 7.91 |
| | | Difference in the measured values (With agitation - without agitation) | 0.00 | 0.12 | 0.12 | -0.11 | 0.14 |
| | No additional (Control) | With agitation before measurement^{*1} | 7.95 | 8.08 | 7.98 | 8.06 | 7.91 |
| | | Without agitation | 7.95 | 6.95 | 6.11 | 5.19 | 5.26 |
| | | Difference in the measured values (With agitation - without agitation) | 0.00 | 1.13 | 1.87 | 2.87 | 2.65 |

| | | | | | | | |
|---|---|---|---|---|---|---|---|
| *1···A second reagent is agitated before measuring. | | | | | | | |

### (2) Measurement of a sample (sample 2) with cystatin C at approximately 4 mg/L

Using a sample (sample 2) with cystatin C at approximately 4 mg/L in place of sample 1, the absorbance change was determined and a measured value of the cystatin C concentration was calculated in the same manner as in (1) of Example 7. The results are shown in Table 9 and in Fig. 1.

**Table 9**

| Sample | Second reagent | | Measured value of cystatin C concentration (mg/L) | | | | |
|---|---|---|---|---|---|---|---|
| | Precipitation preventing substance | Condition | Immediately after application | After two days | After three days | After six days | After seven days |
| Sample 2 (Cystatin C: 4mg/L) | Containing dextran sulfate at 1.0% | With agitation before measurement^{*1} | 3.96 | 4.03 | 4.02 | 3.96 | 3.96 |
| | | Without agitation | 3.96 | 3.94 | 3.97 | 3.96 | 3.97 |
| | | Difference in the measured values (With agitation **-** without agitation) | 0.00 | 0.09 | 0.05 | 0.00 | -0.01 |
| | No additional (Control) | With agitation before measurement^{*1} | 4.00 | 4.09 | 4.00 | 4.09 | 4.07 |
| | | Without agitation | 4.00 | 3.75 | 3.54 | 3.12 | 3.16 |
| | | Difference in the measured values (With agitation - without agitation) | 0.00 | 0.34 | 0.46 | 0.97 | 0.91 |

| | | | | | | | |
|---|---|---|---|---|---|---|---|
| *1...A second reagent is agitated before measuring. | | | | | | | |

It is clear from the results in Tables 8 and 9 and Fig. 1 that using a second reagent to which a precipitation preventing substance (dextran sulfate sodium) was added, stably measured values were obtained up to seven days after without agitation, consistent with measured values with agitation. In contrast, using a second reagent to which no precipitation preventing substance was added, measured values were decreased over time without agitation. Unless agitation is performed before measuring in order to make the concentration of the antibody-bound colloidal gold particles uniform, the antibody-bound colloidal gold particles would gradually precipitate and the concentration of the antibody-bound colloidal gold particles would become diluted at the upper portion of the reagent. As a result, the amount of antibody-bound colloidal gold particles to be added to a reaction system would be decreased.

From this it was found that by adding a precipitation preventing substance (dextran sulfate sodium) to the antibody-bound colloidal gold reagent, precipitation of the antibody-bound colloidal gold particles could be prevented and the concentration of the antibody-bound colloidal gold particles could be uniformly present in the reagent over an extended period of time. Thus, using a second reagent to which a precipitation preventing substance (dextran sulfate sodium) has been added, stably measured values can be obtained without agitating while the reagent is applied to the automated analyzer.

### Example 9: Preparation of a first reagent for measuring ferritin

A first reagent for measuring ferritin was prepared by adding about 1.5 to 2.0% polyethylene glycol as a reaction enhancer to a solution containing 5% sodium chloride, 0.2% EDTA, and 0.35% polyoxyethylene lauryl ether in 0.5 M PIPES (pH 6.5).

### Example 10: Preparation of anti-ferritin antibody bound colloidal gold reagent (Second reagent for measuring ferritin)

Anti-ferritin antibody (Dako Japan) was diluted with 10 mM HEPES (pH 7.1), containing 0.05% sodium azide to obtain a solution that contains 50 µg/mL of anti-ferritin antibody. Then, 100 mL of the solution was added to approximately 1 L of the colloidal gold solution prepared in Example 1, and then incubated for two hours under refrigeration with agitation. Furthermore, 110 mL of a solution containing 5.46% mannitol, 0.5% BSA, and 0.05% sodium azide in 10 mM HEPES (pH 7.1) was added, and then incubated for 90 minutes at 37°C with agitation. The resultant was centrifuged at 8000 rpm for 40 minutes to remove a supernatant, and then approximately 1 L of solution (solution A) containing 3% mannitol, 0.1% BSA, and 0.05% sodium azide in 5 mM HEPES (pH 7.5) was added, to disperse the antibody-bound colloidal gold particles. Furthermore, the dispersion was centrifuged at 8000 rpm for 40 minutes to remove a supernatant, and then the antibody-bound colloidal gold particles were dispersed with the solution A for a total volume of 280 mL, to obtain an anti-ferritin antibody-bound colloidal gold reagent (second reagent).

### Example 11: Examination of the effect for adding dextran sulfate sodium

### (1) Measurement of a sample (sample 4) with ferritin at approximately 760 ng/L

To the anti-ferritin antibody-bound colloidal gold reagent (second reagent), dextran sulfate sodium was added at a concentration of 0.2% to prepare a 0.2% dextran sulfate-added second reagent.

To a Hitachi 7070 automated analyzer, a sample (sample 4) with ferritin at approximately 760 ng/L, a first reagent prepared in Example 9, and a 0.2% dextran sulfate-added second reagent were applied and immediately after the application the absorbance change was measured under the following condition.

### Measurement condition:

First, 160 µL of the first reagent was added to 10 µL of sample 4 and incubated at 37°C for approximately 5 minutes. Then 80 µL of 0.2% dextran sulfate-added second reagent was added and reacted at 37°C. Subsequently, two-point measurement was performed at a dominant wavelength of 505 nm and a secondary wavelength of 700 nm from photometry points 18 to 31, and the absorbance change between two points was determined. The absorbance change was determined for each of the case in which the second reagent was added without agitation (absorbance change without agitation) and the case in which the second reagent was added with agitation (absorbance change with agitation).

Two days after the application, four days after the application, and seven days after the application, the absorbance changes (for each without agitation and with agitation) were also determined in the same manner as above.

The ferritin concentrations were calculated from the determined absorbance changes for "immediately after application", "after two days", "after four days", and "after seven days" using a calibration curve created with a standard solution with a defined concentration. The results are shown in Table 10.

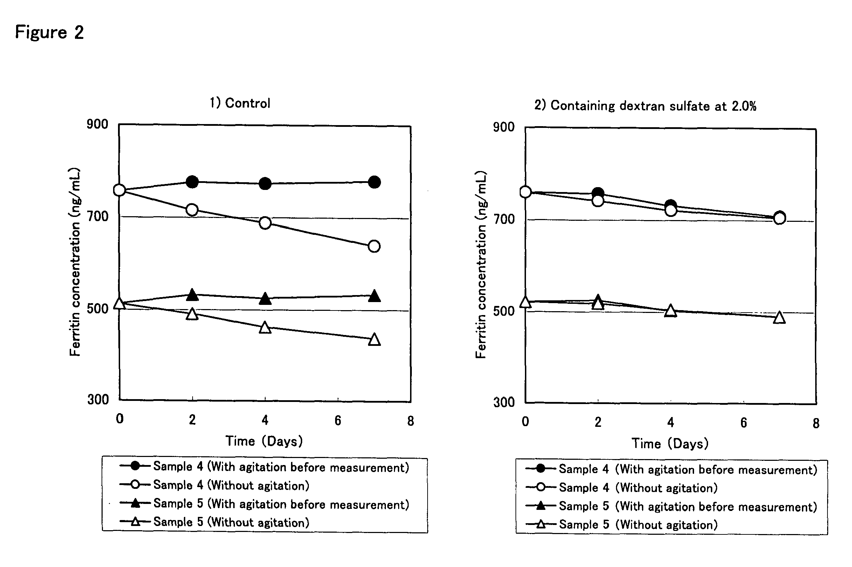

Further, when a second reagent that contains dextran sulfate at 1.0%, a second reagent that contains dextran sulfate at 2.0%, or a second reagent without additional was used in place of the 0.2% dextran sulfate-added second reagent, the absorbance change was determined and a measured value of the ferritin concentration was calculated in the same manner as above. These results also are shown in Table 10. It should be noted that the ferritin concentrations from measurement using a second reagent that contains dextran sulfate at 2.0% and a second reagent without additional (control) are shown together in Fig. 2, respectively.

**Table 10**

| Sample | Second reagent | | Measured value of ferritin concentration (ng/mL) | | | |
|---|---|---|---|---|---|---|
| | Precipitation preventing substance | Condition | Immediately after application | After two days | After four days | After seven days |
| Sample 4 (Ferritin: 760ng/mL) | Containing dextran sulfate at 0.2% | With agitation before measurement^{*1} | 753 | 779 | 776 | 778 |
| | | Without agitation | 753 | 744 | 720 | 698 |
| | | Difference in the measured values (With agitation - without agitation) | 0 | 35 | 56 | 80 |
| | Containing dextran sulfate at 1.0% | With agitation before measurement^{*1} | 762 | 769 | 764 | 769 |
| | | Without agitation | 762 | 774 | 760 | 763 |
| | | Difference in the measured values (With agitation - without agitation) | 0 | -5 | 4 | 6 |
| | Containing dextran sulfate at 2.0% | With agitation before measurement^{*1} | 760 | 758 | 732 | 709 |
| | | Without agitation | 760 | 742 | 722 | 706 |
| | | Difference in the measured values (With agitation - without agitation) | 0 | 16 | 10 | 3 |
| | No additional (Control) | With agitation before measurement^{*1} | 757 | 777 | 774 | 779 |
| | | Without agitation | 757 | 716 | 689 | 640 |
| | | Difference in the measured values (With agitation - without agitation) | 0 | 61 | 85 | 139 |

| | | | | | | |
|---|---|---|---|---|---|---|
| *1···A second reagent is agitated before measuring. | | | | | | |

### (2) Measurement of a sample (sample 5) with ferritin at approximately 520 ng/L

Using a sample (sample 5) with ferritin at approximately 520 ng/L in place of sample 4, the absorbance change was determined and the ferritin concentration was calculated in the same manner as in (1) of Example 11. The results are shown in Table 11. It should be noted that the ferritin concentrations from measurement using a second reagent that contains dextran sulfate at 2.0%, and a second reagent without additional (control) are shown in Fig. 2, respectively.

**Table 11**

| Sample | Second reagent | | Measured value of ferritin concentration (ng/mL) | | | |
|---|---|---|---|---|---|---|
| | Precipitation preventing substance | Condition | Immediately after application | After two days | After four days | After seven days |
| Sample 5 (Ferritin: 520ng/mL) | Containing dextran sulfate at 0.2% | With agitation before measurement^{*1} | 514 | 528 | 527 | 529 |
| | | Without agitation | 514 | 511 | 494 | 477 |
| | | Difference in the measured values (With agitation - without agitation) | 0 | 17 | 33 | 52 |
| | Containing dextran sulfate at 1.0% | With agitation before measurement^{*1} | 523 | 530 | 526 | 529 |
| | | Without agitation | 523 | 530 | 524 | 525 |
| | | Difference in the measured values (With agitation - without agitation) | 0 | 0 | 2 | 4 |
| | Containing dextran sulfate at 2.0% | With agitation before measurement^{*1} | 522 | 526 | 503 | 490 |
| | | Without agitation | 522 | 519 | 505 | 490 |
| | | Difference in the measured values (With agitation - without agitation) | 0 | 7 | -2 | 0 |
| | No additional (Control) | With agitation before measurement^{*1} | 513 | 533 | 526 | 533 |
| | | Without agitation | 513 | 491 | 462 | 437 |
| | | Difference in the measured values (With agitation - without agitation) | 0 | 42 | 64 | 96 |

| | | | | | | |
|---|---|---|---|---|---|---|
| *1···A second reagent is agitated before measuring. | | | | | | |

It is clear from the results in Tables 10 and 11, and Fig. 2, that a second reagent to which a precipitation preventing substance (dextran sulfate sodium) was added had a significantly smaller difference in measured values after two days, four days, and seven days, compared to that to which no precipitation preventing substance was added. Particularly, using a 1.0% dextran sulfate-added second reagent or a 2.0% dextran sulfate-added second reagent, measured values were obtained after seven days without agitation, consistent with measured value with agitation. In contrast, using a second reagent to which no precipitation preventing substance was added, measured values were decreased over time without agitation. Unless agitation is performed before measuring in order to make the concentration of the antibody-bound colloidal gold particles uniform, the antibody-bound colloidal gold particles would gradually precipitate and the concentration of the antibody-bound colloidal gold particles would become diluted at the upper portion of the reagent. As a result, the amount of antibody-bound colloidal gold particles to be added to a reaction system would be decreased.

From this it was found that by adding a precipitation preventing substance (dextran sulfate sodium) to the antibody-bound colloidal gold reagent, precipitation of the antibody-bound colloidal gold particles could be prevented and the concentration of the antibody-bound colloidal gold particles could be uniformly present in the reagent over an extended period of time. Thus, using a second reagent to which a precipitation preventing substance (dextran sulfate sodium) has been added, stably measured values can be obtained without agitating while the reagent is applied to the automated analyzer.

### Industrial Applicability

According to the present invention, precipitation of a reactive substance-bound microparticle can be prevented. Therefore, a reagent in which precipitation of the microparticle is prevented can be provided. In the reagent, the concentration of the microparticle can be uniformly present over an extended period of time. Thus, for example, if the reagent is used for an immunoassay, then reactive substance-bound microparticles therein can be added uniformly to final reaction mixture without agitating the reagent on measuring. Therefore, stably measured values with little error can be provided. Thus, it is possible to not only eliminate necessity for any complicated agitation operations, which imposed a large burden on user, but also prevent measurement error and mistake of clinical decisions due to failing to perform the agitation operation. The reagent and a reagent kit that includes the reagent are useful for automated immuno-analyzer in particular.

The invention may be embodied in other forms without departing from the spirit or essential characteristics thereof. The embodiments disclosed in this specification are to be considered in all respects as illustrative and not limiting. The scope of the invention is indicated by the appended claims rather than by the foregoing description, and all changes which come within the meaning and range of equivalency of the claims are intended to be embraced therein.

## Claims

1. A method of preventing precipitation of a reactive substance-bound microparticle, comprising:
coexisting at least one selected from the group consisting of polyanion or its salt, dextran, cyclodextrin, polyethylene glycol, and glycerol with the microparticle in a dispersion liquid.

2. The method of claim 1, wherein the dispersion liquid contains polyanion or its salt at 0.3 to 5 percent by weight, dextran at 0.1 to 5 percent by weight, cyclodextrin at 0.1 to 5 percent by weight, polyethylene glycol at 0.3 to 5 percent by weight, or glycerol at 5 to 40 percent by weight.

3. The method of claim 1 or 2, wherein the polyanion or its salt is a water-soluble, sulfate group-containing polymer compound.

4. The method of claim 3, wherein the polyanion is dextran sulfate or heparin.

5. The method of any one of claims 1 to 4, wherein the reactive substance-bound microparticle is a colloidal gold particle to which an antibody or antigen is bound.

6. A reagent comprising a reactive substance-bound microparticle and at least one compound selected from the group consisting of polyanion or its salt, dextran, cyclodextrin, polyethylene glycol, and glycerol,
wherein precipitation of the microparticle is prevented.

7. The reagent of claim 6, wherein the reagent contains polyanion or its salt at 0.3 to 5 percent by weight, dextran at 0.3 to 5 percent by weight, cyclodextrin at 0.1 to 5 percent by weight, polyethylene glycol at 0.5 to 5 percent by weight, or glycerol at 5 to 40 percent by weight.

8. The reagent of claim 6 or 7, wherein the polyanion or its salt is a water-soluble, sulfate group-containing polymer compound.

9. The reagent of claim 8, wherein the polyanion is dextran sulfate or heparin.

10. The reagent of any one of claims 6 to 9, wherein the reactive substance-bound microparticle is a colloidal gold particle to which an antibody or antigen is bound.

11. A reagent kit, comprising the reagent of any one of claims 6 to 10.

12. An automated immunoassay method, comprising combining the reagent according to any one of claims 6 to 10 with a sample.
